# EUROPEAN PATENT APPLICATION

(11) **EP 4 403 550 A1**
(43) Date of publication of application: **24.07.2024**
(21) Application number: 22869427.9
(22) Date of filing: 16.09.2022
(51) Int. Cl.: C07D 239/70, C07D 487/08, C07D 401/14, A61K 31/517, A61P 35/00

(54) **FUSED BICYCLIC DERIVATIVE, PHARMACEUTICALLY ACCEPTABLE SALT, CRYSTAL FORM THEREOF AND PREPARATION METHOD THEREFOR**

(30) Priority: 17.09.2021 CN 202111089990
(71) Applicant: Jiangsu Hengrui Pharmaceuticals Co., Ltd., Lianyungang, Jiangsu 222047 (CN); Shanghai Hengrui Pharmaceutical Co., Ltd, Shanghai 200245 (CN)
(72) Inventor: SHANG, Tingting, Lianyungang, Jiangsu 222047 (CN); ZHAO, Miaomiao, Lianyungang, Jiangsu 222047 (CN); YANG, Junran, Lianyungang, Jiangsu 222047 (CN); DU, Zhenxing, Lianyungang, Jiangsu 222047 (CN); WANG, Lin, Lianyungang, Jiangsu 222047 (CN); SHAO, Qiyun, Shanghai 200245 (CN); FENG, Jun, Shanghai 200245 (CN); HE, Feng, Shanghai 200245 (CN)
(74) Representative: dompatent von Kreisler Selting Werner - Partnerschaft von Patent- und Rechtsanwälten mbB
(86) International application number: PCT/CN2022/119402
(87) International publication number: WO 2023/041061

(57) **Abstract**

Provided are a fused bicyclic derivative, a pharmaceutically acceptable salt, a crystal form thereof and a preparation method therefor. Specifically, provided are a hydrochloride, a mesylate, an acetate and a tartrate of the compound of formula (I), and a preparation method therefor and a crystal form thereof.

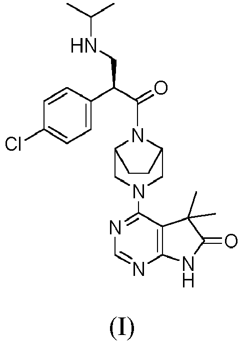

## Description

The present application claims the right of the priority of Chinese patent application 2021110899907 filed on September 17, 2021. The content of the above Chinese patent application is incorporated herein by reference in its entirety.

### TECHNICAL FIELD

The present disclosure belongs to the technical field of medicine and relates to a pharmaceutically acceptable salt and crystal form of a fused bicyclic derivative, and a preparation method therefor.

### BACKGROUND

Protein kinase B (PKB, also known as AKT) is central to PI3K/AKT/mTOR signaling in cells, and its function has important roles in cell growth, survival, differentiation, and metabolism. The PI3K signaling pathway is involved in and regulates the expression of multiple oncogenes and anticancer genes, and over-activation of the PI3K/AKT signaling pathway has been proved to be associated with the development of multiple cancers.

In cells, AKT can be activated by a series of signals, including growth factors. When the receptor tyrosine kinase on the cell membrane is activated by a growth factor, downstream PI3K is activated, so that phosphatidylinositol-4,5-biphosphate (PIP2) is phosphorylated to form phosphatidylinositol-3,4,5-triphosphate (PIP3). Finally, phosphatidylinositol-dependent kinase 1 (PDK1) and AKT are recruited to the cell membrane, and then AKT is activated by PDK1. Variation in PI3K and deletion and variation in PTEN will continuously activate AKT protein, which results in continuous activation of this pathway. The main function of AKT in cells is to promote cell proliferation, cause the cells to be transformed from benign ones to malignant ones and promote cell movement and invasion, thereby causing the metastasis and dissemination of tumor cells; besides, the high-activity phosphorylated AKT can also inhibit apoptosis and participate in chemotherapy resistance mechanism and thereby influence the effect of clinical treatment. In clinical statistics, the proportion of tumors with high-activity AKT in each different tumor can reach 40% or more.

There are 3 subtypes of AKT (AKT1, AKT2, and AKT3), each of which has been shown to function differently *in vivo* in various studies. Signal pathways activated by AKT1 mainly regulate cell proliferation and survival, and AKT2 has such functions as participating in cell invasion and migration and insulin-regulated blood sugar metabolism pathway. Although the gene knockout mouse of AKT3 only shows functions related to embryonic brain development, the clinical research shows that the expression level of AKT3 is increased significantly in various tumors, such as breast cancer. In addition, *in vitro* studies before clinic use show that the breast cancer cells can generate drug resistance in the long-term treatment with an AKT1/2 selective inhibitor MK2206, and the expression level of AKT3 is increased remarkably in the drug resistant cells.

Inhibitors targeting AKT have been studied clinically for many years. Disclosed patent applications of AKT inhibitors include WO2006/071819, U.S. Pat. No. 8,377,937, WO2008/075109, US2010120801, and WO2009006040. The selective inhibitors of AKT1/2, MK2206 (Merck) and BAY1125976 (Bayer), have not been clinically successful for reasons related to therapeutic effects, toxicity and the like. However, in recent years, AKT1/2/3(AKT pan) inhibitors AZD5363 (AZ) and GDC0068 (Roche) have made breakthroughs in phase 2 clinical trial, and their combination with other anticancer drugs has exhibited significant efficacy in the treatment of triple negative breast cancer, ER+ breast cancer, and prostate cancer. At present, the two AKT 1/2/3 (AKT pan) inhibitors AZD5363 and GDC0068 have successfully entered the phase 3 clinical trial.

Application PCT/CN2021/081033 discloses a compound of formula I. In order to meet the needs of drug development, it is necessary to study its pharmaceutically acceptable salt and corresponding crystal form.

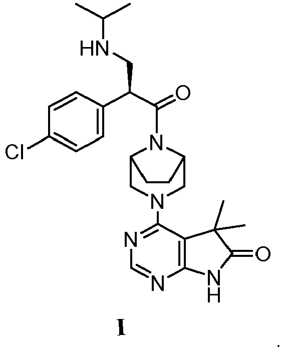

### CONTENT OF THE PRESENT INVENTION

The present disclosure provides a pharmaceutically acceptable salt of a compound of formula I, wherein the pharmaceutically acceptable salt is selected from hydrochloride, methanesulfonate, acetate, or tartrate,

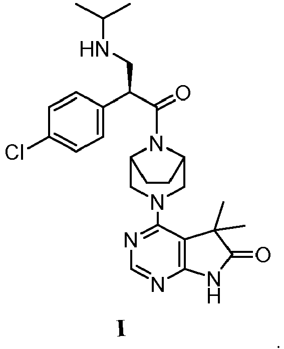

In an optional embodiment, the molar ratio of the compound of formula I to the acid radical is selected from 1:2 to 3:1, preferably 1:1 and 2:1.

The present disclosure also provides a method for preparing the pharmaceutically acceptable salt of the compound of formula I, which comprises a salt-forming step with the compound of formula I and an acid. In an optional embodiment, the solvent used in the salt-forming reaction is selected from at least one of isopropanol, acetonitrile, ethanol, water, and isopropyl acetate. In an optional embodiment, the method for preparing the foregoing pharmaceutically acceptable salt also comprises steps such as volatilizing the solvent or stirring for crystallization, filtering, and drying.

The present disclosure provides a crystal form A of hydrochloride of the compound of formula I, which has an X-ray powder diffraction pattern comprising characteristic peaks at 2θ angles of 10.6, 11.4, 13.6, 17.6, 18.2, and 18.8.

In an optional embodiment, the crystal form A of the hydrochloride of the compound of formula I has the X-ray powder diffraction pattern comprising characteristic peaks at 2θ angles of 10.6, 11.4, 13.6, 15.0, 17.1, 17.6, 18.2, 18.8, 20.5, 22.0, and 27.5.

In an optional embodiment, the crystal form A of the hydrochloride of the compound of formula I has the X-ray powder diffraction pattern comprising characteristic peaks at 2θ angles of 10.6, 11.0, 11.4, 12.3, 13.6, 14.3, 15.0, 17.1, 17.6, 18.2, 18.8, 20.2, 20.5, 21.3, 22.0, 23.0, 23.7, 24.4, 25.0, 25.4, 26.1, 26.6, 27.5, 28.3, 28.7, 29.4, 30.4, 31.4, 31.9, 32.2, 33.3, 34.9, 38.1, 39.9, and 41.0.

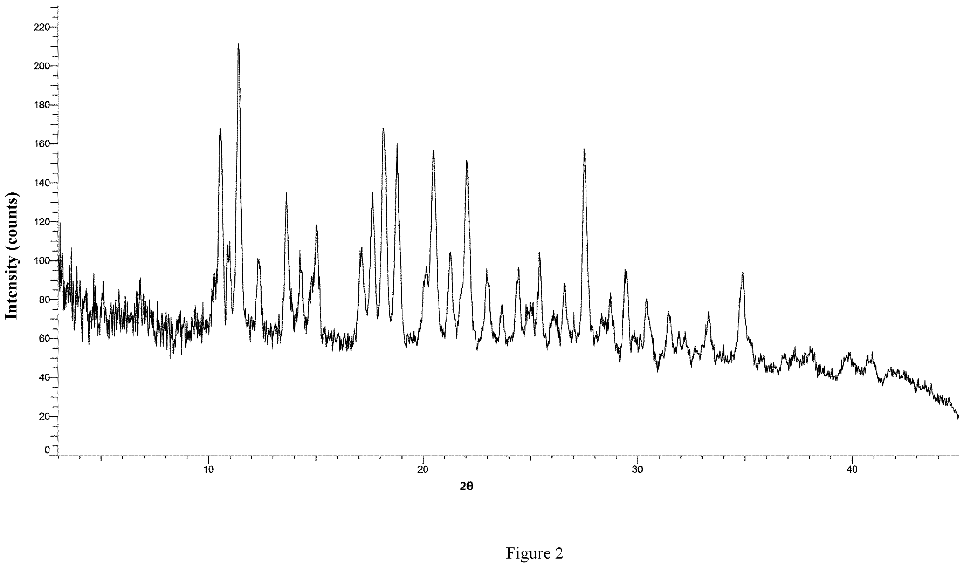

In an optional embodiment, the crystal form A of the hydrochloride of the compound of formula I has the X-ray powder diffraction pattern as shown in Figure 2.

The present disclosure further provides a method for preparing the crystal form A of the hydrochloride of the compound of formula I, which comprises: mixing the compound of formula I with an appropriate amount of a solvent and hydrochloric acid, and stirring for crystallization, the solvent being one or more than one of isopropanol, acetonitrile, water/ethanol, and isopropyl acetate.

The present disclosure also provides a crystal form A of methanesulfonate of the compound of formula I, which has an X-ray powder diffraction pattern comprising characteristic peaks at 2θ angles of 9.1, 11.0, 14.9, 16.2, and 17.0.

In an optional embodiment, the crystal form A of the methanesulfonate of the compound of formula I has the X-ray powder diffraction pattern comprising characteristic peaks at 2θ angles of 9.1, 11.0, 14.9, 16.2, 17.0, 20.1, 20.9, 21.2, 24.3, and 27.4.

In an optional embodiment, the crystal form A of the hydrochloride of the compound of formula I has the X-ray powder diffraction pattern comprising characteristic peaks at 2θ angles of 9.1, 10.1, 11.0, 12.2, 13.6, 14.9, 15.4, 15.8, 16.2, 17.0, 18.0, 18.6, 19.3, 20.1, 20.9, 21.2, 22.2, 23.1, 23.6, 24.3, 24.5, 24.9, 25.0, 25.2, 26.0, 27.4, 27.7, 28.8, 29.4, 29.8, 30.2, 30.8, 31.3, 32.1, 32.8, and 33.8.

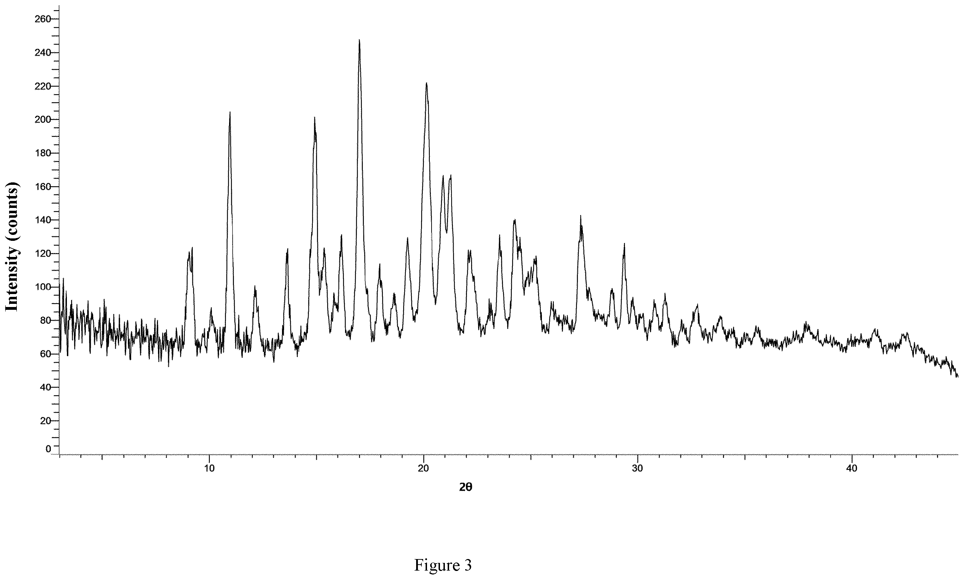

In an optional embodiment, the crystal form A of the methanesulfonate of the compound of formula I has the X-ray powder diffraction pattern as shown in Figure 3.

The present disclosure further provides a method for preparing the crystal form A of the methanesulfonate of the compound of formula I, which comprises: mixing the compound of formula I with an appropriate amount of a solvent and methanesulfonic acid, and stirring for crystallization, the solvent being one or more than one of isopropanol, acetonitrile, ethanol, water/ethanol, and isopropyl acetate.

The present disclosure also provides a crystal form α of acetate of the compound of formula I, which has an X-ray powder diffraction pattern comprising characteristic peaks at 2θ angles of 8.9, 11.0, 14.9, 15.7, 17.0, and 19.7.

In an optional embodiment, the crystal form α of the acetate of the compound of formula I has the X-ray powder diffraction pattern comprising characteristic peaks at 2θ angles of 8.9, 11.0, 14.9, 15.7, 17.0, 19.1, 19.7, 20.4, 23.6, 23.9, and 27.2.

In an optional embodiment, the crystal form α of the acetate of the compound of formula I has the X-ray powder diffraction pattern comprising characteristic peaks at 2θ angles of 8.9, 10.3, 11.0, 13.6, 14.9, 15.7, 17.0, 18.1, 18.6, 19.1, 19.7, 20.4, 20.8, 21.3, 22.2, 23.6, 23.9, 24.9, 26.0, 27.2, 28.3, 28.7, 29.0, 30.8, 32.4, 33.6, 33.9, 34.5, 35.3, 36.4, 37.0, 37.4, and 38.4.

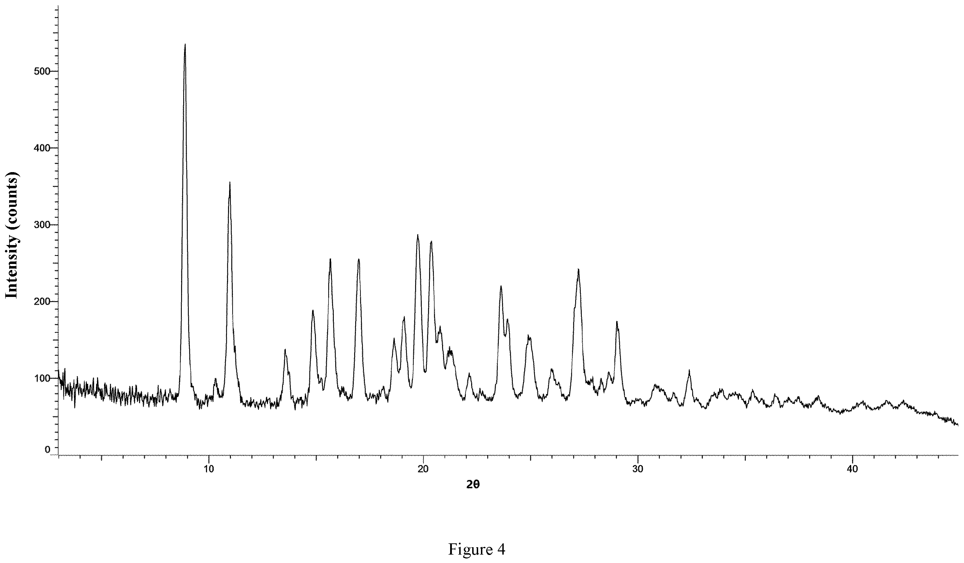

In an optional embodiment, the crystal form α of the acetate of the compound of formula I has the X-ray powder diffraction pattern as shown in Figure 4.

The present disclosure further provides a method for preparing the crystal form α of the acetate of the compound of formula I, which comprises: mixing the compound of formula I with an appropriate amount of a solvent and acetic acid, and stirring for crystallization, the solvent being one or more than one of isopropanol, acetonitrile, and water/ethanol.

The present disclosure also provides a crystal form I of tartrate of the compound of formula I, which has an X-ray powder diffraction pattern comprising characteristic peaks at 7.9, 8.6, 10.0, 10.9, 11.7, 13.9, 14.2, 14.4, 15.3, 16.4, 17.4, 17.8, 19.0, 20.1, 21.2, 21.5, 22.3, 23.7, 24.0, 24.6, 25.1, 26.3, 27.7, 28.7, 29.3, 30.6, and 30.9.

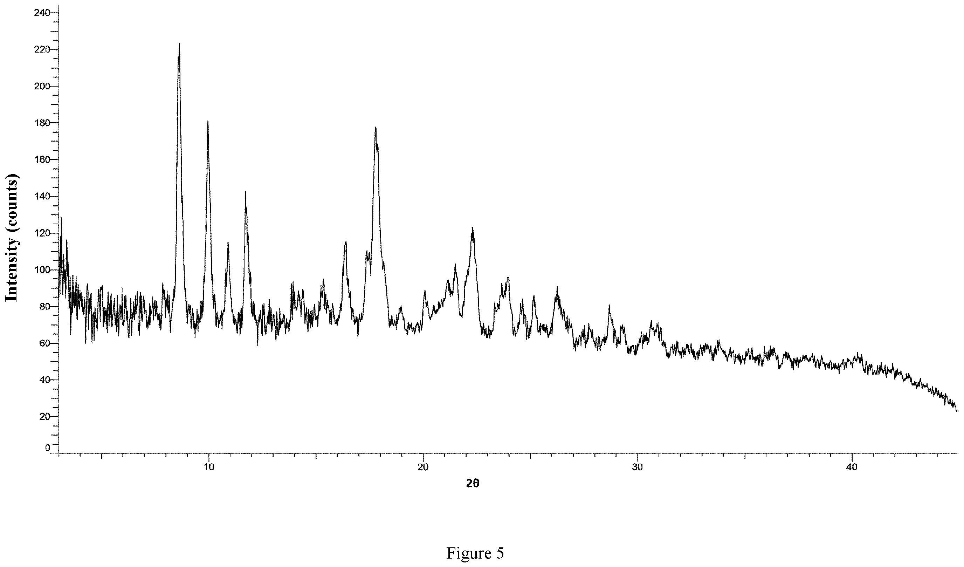

In an optional embodiment, the crystal form I of the tartrate of the compound of formula I has the X-ray powder diffraction pattern as shown in Figure 5.

The present disclosure further provides a method for preparing the crystal form I of the tartrate of the compound of formula I, which comprises: mixing the compound of formula I with an appropriate amount of a solvent and tartaric acid, and stirring for crystallization, the solvent being one or more than one of isopropanol and water/ethanol.

TThe structure determination and crystal form study of the crystal form obtained in the present disclosure are carried out through X-ray powder diffraction (XRPD) and differential scanning calorimetry (DSC).

The crystallization methods of the crystal forms in the present disclosure are conventional, such as volatilization crystallization, cooling crystallization, or crystallization at room temperature.

The starting raw material used in the method for preparing the salt or crystal form of the present disclosure can be any form of the compound of formula I, and specific forms include but are not limited to: amorphous, any crystal form, hydrate, solvate, etc.

The present disclosure further provides a pharmaceutical composition comprising the following components: (a) the pharmaceutically acceptable salt of the compound of formula I or the crystal form of the pharmaceutically acceptable salt of the compound of formula I; and (b) an optional pharmaceutically acceptable carrier, diluent, or excipient.

The present disclosure also provides a method for preparing the pharmaceutical composition, comprising a step of mixing: (a) the pharmaceutically acceptable salt of the compound of formula I or the crystal form of the pharmaceutically acceptable salt of the compound of formula I; and (b) the optional pharmaceutically acceptable carrier, diluent, or excipient.

The present disclosure further provides a use of the foregoing pharmaceutically acceptable salt of the compound of formula I, the crystal form of the pharmaceutically acceptable salt of the compound of formula I, or the composition in the manufacture of a medicament for treating and/or preventing cancer, wherein the cancer is preferably selected from ovarian cancer, breast cancer, prostate cancer, neuroglioma, spongiocytoma, gastric cancer, fallopian tube cancer, lung cancer, peritoneal tumor, melanoma, brain cancer, esophageal cancer, liver cancer, pancreatic cancer, colorectal cancer, lung cancer, kidney cancer, cervical cancer, skin cancer, neuroblastoma, sarcoma, bone cancer, uterine cancer, endometrial cancer, head and neck tumor, multiple myeloma, lymphoma, non-Hodgkin's lymphoma, non-small cell lung cancer, polycythemia vera, leukemia, thyroid tumor, bladder cancer, and gallbladder cancer.

In the specification and claims of the present disclosure, unless otherwise specified, scientific and technical terms used herein have the meanings commonly understood by those skilled in the art. However, in order to better understand the present disclosure, definitions and explanations of some relevant terms are provided below. In addition, when the definitions and explanations of terms provided in the present disclosure are inconsistent with the meanings commonly understood by those skilled in the art, the definitions and explanations of terms provided in the present disclosure shall prevail.

The "X-ray powder diffraction pattern or XRPD" described in the present disclosure refers to the group of X-ray powder diffraction patterns measured by satisfying the Bragg equation according to the Bragg formula 2d sin θ = nλ (where λ is the wavelength of X-rays, the diffraction order n is any positive integer, generally taking the first order diffraction peak, n = 1), when X-rays incident at a glancing angle θ (the complement of the incident angle, also known as the Bragg angle) on a certain atomic plane of a crystal or partial crystal sample with a lattice plane spacing of d.

The "X-ray powder diffraction pattern or XRPD" described in the present disclosure is a pattern obtained by using Cu-Kα radiation in an X-ray powder diffractometer.

The "differential scanning calorimetry or DSC" described in the present disclosure refers to measuring the temperature difference and heat flow difference between the sample and the reference during the process of heating or constant temperature of the sample, so as to characterize all physical changes and chemical changes related to thermal effects and obtain the phase change information of the sample.

The "thermogravimetric analysis or TGA" described in the present disclosure refers to the continuous measurement of the mass change of the sample with temperature or time under program-controlled temperature.

The "2θ or 2θ angle" described in the present disclosure refers to the diffraction angle, θ is the Bragg angle with a unit of° or degrees, and the 2θ has an error range of ± 0.3 or ± 0.2 or ± 0.1.

The "interplanar spacing or d-spacing (d value)" described in the present disclosure refers to the point lattice selecting three non-parallel unit vectors a, b, c connecting adjacent two lattice points, which divide the point lattice into juxtaposed parallelepiped units, called interplanar spacing. The point lattice is divided according to the determined lines connecting parallelepiped units to obtain a set of straight-line grids, which are called space lattice or crystal lattice. Point lattice and crystal lattice use geometric points and lines to reflect the periodicity of the crystal structure respectively. Different crystal planes have different interplanar spacings (that is, the distance between two adjacent parallel crystal planes); the unit is Å or angstrom.

### BRIEF DESCRIPTION OF THE DRAWINGS

Figure 1 is the XRPD pattern of an amorphous form of the compound of formula I;
Figure 2 is the XRPD pattern of the crystal form A of the hydrochloride of the compound of formula I in Example 2;
Figure 3 is the XRPD pattern of the crystal form A of the methanesulfonate of the compound of formula I in Example 6;
Figure 4 is the XRPD pattern of the crystal form α of the acetate of the compound of formula I in Example 10;
Figure 5 is the XRPD pattern of the crystal form I of the tartrate of the compound of formula I in Example 13;
Figure 6 is the DSC pattern of the crystal form A of the hydrochloride of the compound of formula I in Example 2;
Figure 7 is the DSC pattern of the crystal form A of the methanesulfonate of the compound of formula I in Example 6;
Figure 8 is the DSC pattern of the crystal form α of the acetate of the compound of formula I in Example 10;
Figure 9 is the DSC pattern of the crystal form I of the tartrate of the compound of formula I in Example 13.

### DETAILED DESCRIPTION OF THE PREFERRED EMBODIMENT

The present disclosure will be explained in more detail below with reference to examples. The examples of the present disclosure are only used to illustrate the technical solutions of the present disclosure and do not limit the essence and scope of the present disclosure.

Test conditions of the instruments used in the experiment:
The structure of the compound was determined by nuclear magnetic resonance (NMR) spectroscopy and/or mass spectrometry (MS).

NMR shift (δ) is given in a unit of 10⁻⁶ (ppm). NMR spectra were measured using a Bruker AVANCE-400 nuclear magnetic resonance instrument or Bruker AVANCE NEO 500M, with deuterated dimethyl sulfoxide (DMSO-d₆), deuterated chloroform (CDCl₃) and deuterated methanol (CD₃OD) as determination solvents and tetramethylsilane (TMS) as an internal standard.

Mass spectra were measured using Agilent 1200/1290 DAD- 6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector), and THERMO Ultimate 3000-Q Exactive (manufacturer: THERMO, MS model: THERMO Q Exactive).

High performance liquid chromatography (HPLC) was performed using the following HPLC instruments: Agilent HPLC 1200DAD, Agilent HPLC 1200VWD, and Waters HPLC e2695-2489.

Chiral HPLC was performed on Agilent 1260 DAD HPLC.

HPLC preparation was performed using Waters 2767, Waters 2767-SQ Detecor2, Shimadzu LC-20AP, and Gilson-281 preparative chromatographs.

Chiral preparation was performed on a Shimadzu LC-20AP preparative chromatograph.

A CombiFlash Rf200 (TELEDYNE ISCO) system was used for rapid preparation.

XRPD is X-ray powder diffraction detection: The measurement was carried out using a BRUKER D8 X-ray diffractometer. The specific collection information is: monochromatic Cu-Kα ray (λ = 1.5406), voltage: 40 kV, current: 40 mA. Scanning mode: θ/2θ, scanning range (2θ range): 3 to 45°.

DSC is differential scanning calorimetry: The measurement was carried out using a METTLER TOLEDO DSC 3 + differential scanning calorimeter, with a heating rate of 10°C/min, a specific temperature range referring to the corresponding pattern (mostly 25 to 200 or 25 to 300°C), and a nitrogen purging speed of 50 mL/min.

TGA is thermogravimetric analysis: The detection was carried out using a METTLER TOLEDO TGA 2 thermogravimetric analyzer, with a heating rate of 10°C/min, a specific temperature range referring to the corresponding pattern (mostly 25 to 350°C), and a nitrogen purging speed of 50 mL/min.

DVS is dynamic vapor sorption: The detection was carried out using SMS DVS Advantage at 25°C with a humidity change of 50%-95%-0%-95%-50% in steps of 10% (the last step was 5%) (the specific range of humidity was subject to the corresponding pattern, and most methods were listed here), and the judgment standard was that dm/dt was not greater than 0.002%.

Ion chromatography detection: Instrument: American DIONEX INTERGRION ion chromatograph, detection method: conductivity; separation column: IonPac AS27; eluent: 30 mM KOH; flow rate: 1.5 mL/min.

Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

The silica gel column chromatography generally used 200 to 300-mesh silica gel (Huanghai, Yantai) as the carrier.

The mean inhibition of kinase and the IC50 value were measured using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

A hydrogen atmosphere means that the reaction flask is connected to a balloon containing about 1 L of hydrogen.

Parr 3916EKX hydrogenator, Qinglan QL-500 hydrogenator, or HC2-SS hydrogenator was used in the pressurized hydrogenation reactions.

The hydrogenation reactions usually involve 3 cycles of vacuumization and hydrogen purge.

A CEM Discover-S 908860 microwave reactor was used in the microwave reactions.

In the examples, a solution refers to an aqueous solution unless otherwise specified.

In the examples, the reaction temperature was room temperature, i.e., 20°C to 30°C, unless otherwise specified.

The monitoring of the reaction progress in the examples was conducted by thin layer chromatography (TLC). The developing solvent for reactions, the eluent system for column chromatography purification and the developing solvent system for thin layer chromatography included: C: petroleum ether/ethyl acetate system. The volume ratio of the solvents was adjusted according to the polarity of the compound, or by adding a small amount of basic or acidic reagents such as triethylamine and acetic acid.

### Example 1: Preparation of Compound of Formula I

### 4-((1R,5S)-8-(S)-2-(4-Chlorophenyl)-3-(isopropylamino)propanoyl)-3,8-diazabicyclo[3.2.1]octan-3-yl)-5,5-dimethyl-5,7-dihydro-6H-pyrrolo[2,3-d]pyrimidin-6-one I

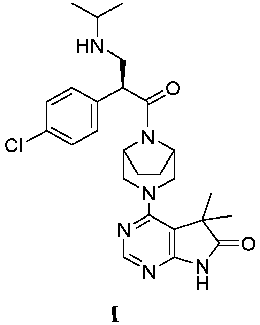

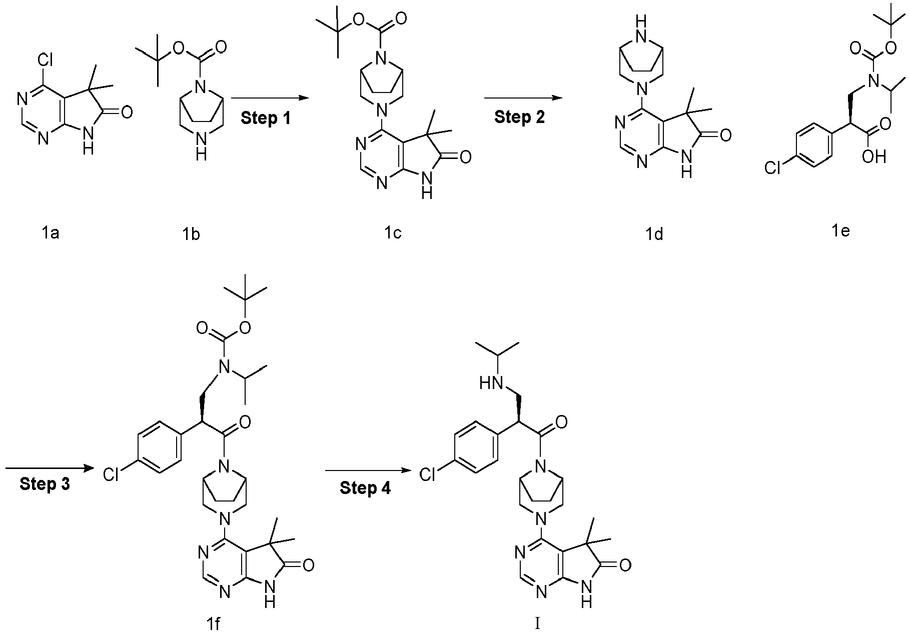

### Step 1

### tert-Butyl (1R,5S)-3-(5,5-dimethyl-6-oxo-6,7-dihydro-5H-pyrrolo[2,3-d]pyrimidin-4-yl)-3,8-diazabicyclo[3.2.1]octane-8-carboxylate 1c

*tert*-Butyl (1*R*,5*S*)-3,8-diazabicyclo[3.2.1]octane-8-carboxylate **1b** (250 mg, 1.18 mmol, Bide Pharmatech CO., Ltd.), 4-chloro-5,5-dimethyl-7*H*-pyrrolo[2,3-*d*]pyrimidin-6-one **1a** (233 mg, 1.18 mmol, Bide Pharmatech CO., Ltd.), and *N*,*N-*diisopropylethylamine (457 mg, 3.53 mmol) were dissolved in *N,N-*dimethylformamide (5 mL), and the reaction solution was stirred at 120°C overnight. The reaction solution was cooled to room temperature and concentrated under reduced pressure, and the residue was purified by column chromatography with a developing solvent system C to obtain the title compound **1c** (220 mg, yield: 50.0%).

MS m/z (ESI): 374.1 [M+1].

### Step 2

### 4-((1R,5S)-3,8-Diazabicyclo[3.2.1]octan-3-yl)-5,5-dimethyl-5,7-dihydro-6H-pyrrolo[2,3-d]pyrimidin-6-one 1d

Compound **1c** was dissolved in a solution of hydrogen chloride in dioxane (4 M, 5 mL) and stirred at room temperature for 1 h. The reaction solution was concentrated under reduced pressure to obtain the title compound **1d** (crude product), which was used directly in the next step.

MS m/z (ESI): 274.1 [M+1].

### Step 3

### tert-Butyl ((S)-2-(4-chlorophenyl)-3-((1R,5S)-3-(5,5-dimethyl-6-oxo-6,7-dihydro-5H-pyrrolo[2,3-d]pyrimidin-4-yl)-3,8-diazabicyclo[3.2.1]octan-8-yl)-3-oxopropyl)(isopropyl)carbamate 1f

Compound **1d** (160 mg, 585 µmol) and (*S*)-3-((*tert-*butyloxycarbonyl)(isopropyl)amino)-2-(4-chlorophenyl)propanoic acid **1e** (200 mg, 585 µmol, prepared by the method disclosed in "J. Med. Chem.2012, 55, 8110-8127*")* were dissolved in 5 mL of dichloromethane, and then 2-(7-azabenzotriazol-1-yl)-*N*,*N*,*N*',*N*'-tetramethyluronium hexafluorophosphate (334 mg, 878 µmol) and triethylamine (237 mg, 2.34 mmol) were added. The reaction solution was stirred overnight. The reaction solution was diluted with ethyl acetate (20 mL), washed with water, and concentrated under reduced pressure to obtain the title compound **1f** (crude product, 130 mg), which was used directly in the next step.

MS m/z (ESI): 597.1 [M+1].

### Step 4

### 4-((1R,5S)-8-(S)-2-(4-Chlorophenyl)-3-(isopropylamino)propanoyl)-3,8-diazabicyclo[3.2.1]octan-3-yl)-5,5-dimethyl-5,7-dihydro-6H-pyrrolo[2,3-d]pyrimidin-6-one I

Compound **1f** (crude product) was dissolved in ethyl acetate (2 mL), and a solution of hydrogen chloride in dioxane (4 M, 5 mL) was added with stirring at room temperature, and stirred at room temperature for 1 h. The reaction solution was concentrated under reduced pressure, and the residue was purified by preparative chromatography to obtain the title compound **I** (30 mg, yield: 27.7%).

MS m/z (ESI): 497.2 [M+ 1].

¹H NMR (600 MHz, CD₃OD): δ 8.21 (d, 1H), 7.41-7.35 (m, 4H), 4.83-4.82 (m, 1H), 4.56-4.43 (m, 2H), 4.34-4.27 (m, 2H), 4.17-4.09 (m, 1H), 3.90-3.87 (m, 1H), 2.99-2.97 (m, 1H), 2.91-2.87 (m, 1H), 2.82-2.77 (m, 1H), 2.11-2.07 (m, 1H), 2.01-1.99 (m, 1H), 1.96-1.90 (m, 1H), 1.86-1.80 (m, 1H), 1.74-1.66 (m, 2H), 1.51-1.50 (m, 2H), 1.41 (s, 2H), 1.32 (s, 2H), 1.13-1.09 (m, 6H).

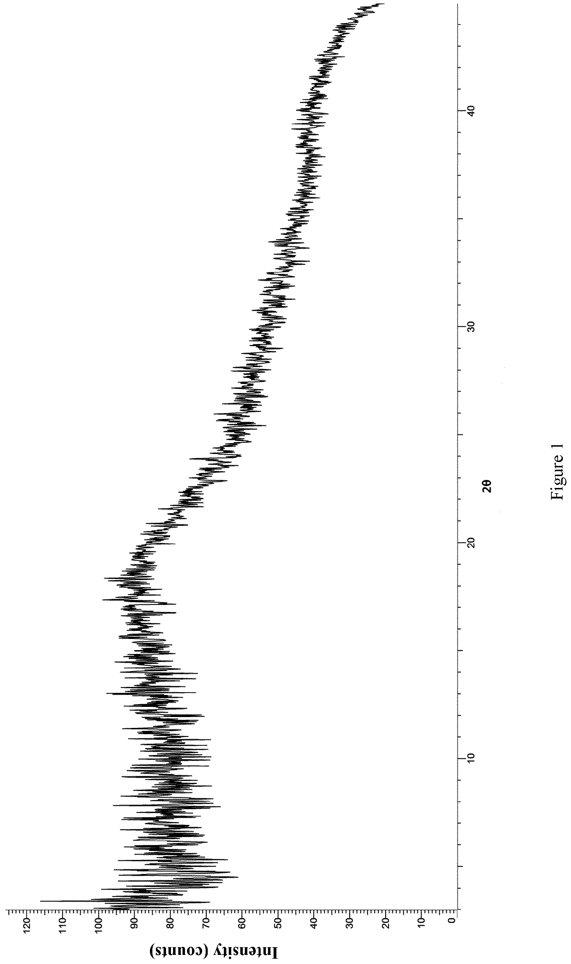

The product was detected as amorphous by X-ray powder diffraction and its XRPD pattern is shown in Figure 1.

### Test Example 1: Biological Evaluation

The following methods were used to determine the inhibitory effect of the compound of formula Ion the kinase activity of AKT1/AKT2/AKT3 *in vitro.* The experimental method is briefly described as follows:
The enzyme activity of AKT1 (Invitrogen, P2999), AKT2 (Invitrogen, PV3184), and AKT3 (Invitrogen, PV3185) was determined using a KinEASE-STK S3 kit (Cisbio, 62ST3PEC). Test compounds were each first subjected to 3-fold gradient dilution with DMSO from 500 µM to obtain a total of 11 concentrations. The 5× buffer in the kit was diluted to 1× buffer, and DTT (Sigma, 43816-10ML) and MgCl₂ were added to make the buffer contain 1 mM DTT and 5 mM MgCl₂. Compounds were each subjected to 20-fold dilution with 1× buffer for later use. The enzyme solution was obtained by diluting the AKT1/AKT2/AKT3 kinase with 1× buffer. ATP (Invitrogen, PV3227) and S3-biotin in the kit were diluted with 1× buffer to obtain a substrate-ATP mixture solution for later use. 2 µL of the enzyme solution and 4 µL of the compound solution were added to each well of a 384-well plate (Corning, 4513), and the mixture was incubated at room temperature for 30 min, followed by addition of 4 µL of the ATP and S3-biotin mixture solution. The resulting mixture was incubated at room temperature for 90 min. AKT1 enzyme reaction conditions were a final concentration of 2 nM for enzyme, a final concentration of 10 µM for ATP, and a final concentration of 2 µM for S3-biotin. AKT2 enzyme reaction conditions were a final concentration of 5 nM for enzyme, a final concentration of 10 µM for ATP, and a final concentration of 2 µM for S3-biotin. AKT3 enzyme reaction conditions were a final concentration of 0.4 nM for enzyme, a final concentration of 45 µM for ATP, and a final concentration of 2 µM for S3-biotin. The detection solution was prepared by diluting the S3-cryptate and Streptavidin-XL665 with the detection buffer in the kit. After incubation, 10 µL of the detection solution was added to each well, the final concentration of S3-cryptate was a concentration obtained by 200-fold dilution of the stock solution, and the final concentration of streptavidin-XL665 was 125 nM. The mixture was incubated at room temperature for 60 min, values of signals emitted at 650 nm and 620 nm after excitation at 337 nm were measured by using an HTRF module of a multi-functional microplate detector (BMG Labtech, PHERAstar FS), and the ratio of the readings was multiplied by 10,000 to obtain a ratio value. Dose-response curves were drawn according to the concentration of the compounds and the ratio values by using Graphpad Prism software, and the IC₅₀ values for the inhibitory activity of the compounds were calculated.

### Experimental Data

The inhibitory activity of the compound of formula I against AKT1/AKT2/AKT3 enzymes was determined by the above assay, and the IC₅₀ values measured are shown in Table 1.

**Table 1 IC₅₀ values for AKT1/AKT2/AKT3 enzyme inhibition by the compound of formula I**

| Sample | AKT1 | AKT2 | AKT3 |
|---|---|---|---|
| | IC₅₀/nM | IC₅₀/nM | IC₅₀/nM |
| Compound of formula I | 60.0 | 74.1 | 2.0 |

Conclusion: The compound of formula I has a good inhibitory effect on AKT1/AKT2/AKT3 enzymes.

### Pharmacokinetic Test of Compound of Formula I

### 1. Abstract

The drug concentration in the plasma of the test animals (Balb/c nude mice) at different time points after intragastric administration (i.g.) of the test compounds was determined by using an LC/MS/MS method. The pharmacokinetic performance of the compound of formula I in nude mice was studied and their pharmacokinetic profile was evaluated.

### 2. Test Scheme

### 2.1. Test Compounds

### Compound of formula I.

### 2.2. Test Animals

18 Balb/c nude mice, female, divided into 2 groups and purchased from Vital River Laboratory Animal Technology Co., Ltd.

### 2.3. Drug Preparation

A certain amount of compound was weighed out, and 99.8% (0.5% methylcellulose) was added into the compound, and then 0.2% Tween was added. The mixture was subjected to ultrasonic treatment to obtain a suspension, which was stirred for administration.

### 2.4. Administration

The nude mice were subjected to intragastric administration with a dose of 50 mg/kg or 100 mg/kg and an administration volume of 0.2 mL/10 g.

### 3. Procedures

The test compound was administered intragastrically to the nude mice, and 0.1 mL of blood was collected before administration and 0.25 h, 0.5 h, 1.0 h, 2.0 h, 4.0 h, 6.0 h, 8.0 h, 11.0 h, and 24.0 h after administration. The blood was placed in an EDTA-K2 anticoagulation tube and centrifuged at 10,000 rpm for 1 min (4°C), and plasma was separated out within 1 h and then stored at -20°C for testing. The process from blood sampling to centrifugation was performed under an ice bath.

The content of the test compounds in the nude mice plasma after intragastric administration of different concentrations of the compound was determined: 20 µL of nude mouse plasma at each time point after administration was mixed with 50 µL of internal standard solution (camptothecin, 100 ng/mL) and 200 µL of acetonitrile, and the mixture was vortexed for 5 min and centrifuged for 10 min at 3700 rpm. 1 µL of supernatant of the plasma sample was taken for LC/MS/MS analysis.

### 4. Pharmacokinetic Parameter Results

The results are shown in Table 2.

**Table 2 Pharmacokinetic parameters of compound of formula I**

| Dose (mg/kg) | Plasma concentration | Area under curve | Half-life | Residence time | Clearance rate | Apparent volume of distribution |
|---|---|---|---|---|---|---|
| | Cmax (ng/mL) | AUC₀₋ₜ (ng /mL*h) | T_{1/2} (h) | MRT (h) | CL/F (mL/min/k g) | Vz/F (mL/kg) |
| 50 | 3000 | 20207 | 2.3 | 4.9 | 41.2 | 8029 |
| 100 | 3500 | 35990 | 4.1 | 6.7 | 45.4 | 16213 |

Conclusion: The compound of formula I has good pharmacokinetic absorption, and as the dose increases, the absorption increases accordingly.

### Pharmacokinetic Test of Compound of Formula I in Rats

### 1. Abstract

The drug concentration in the plasma of the test animals (SD rats) at different time points after injection (i.v.) of the test compounds was determined by using an LC/MS/MS method. The pharmacokinetic performance of the compound of formula I in rats was studied and their pharmacokinetic profile was evaluated.

### 2. Test Scheme

### 2.1. Test Compounds

### Compound of formula I.

### 2.2. Test Animals

8 SD rats, half male and half female, divided into 2 groups, purchased from Vital River Laboratory Animal Technology Co., Ltd.

### 2.3. Drug Preparation

A certain amount of the compound was weighed out, and 5% of DMSO, 5% of Tween 80, and 90% of normal saline were added to obtain a colorless and clear solution.

### 2.4. Administration

The rats were subjected to administration by injection with a dose of 1 mg/kg and an administration volume of 5 mL/kg.

### 3. Procedures

The test compound was administered by injection to the rats, and 0.2 mL of blood was collected from the orbit before administration and 5 min, 0.25 h, 0.5 h, 1.0 h, 2.0 h, 4.0 h, 8.0 h, 11.0 h, and 24.0 h after administration. The blood was placed in an EDTA-K2 anticoagulation tube and centrifuged at 10,000 rpm for 1 min (4°C), and plasma was separated out within 1 h and then stored at -20°C for testing. The process from blood sampling to centrifugation was performed under an ice bath.

The content of the test compounds in the rat plasma after administration by injection of different concentrations of the compound was determined: 25 µL of rat plasma at each time point after administration was mixed with 50 µL of internal standard solution (camptothecin, 100 ng/mL) and 200 µL of acetonitrile, and the mixture was vortexed for 5 min and centrifuged for 10 min at 3700 rpm. 3 µL of supernatant of the plasma sample was taken for LC/MS/MS analysis.

### 4. Pharmacokinetic Parameters

The results are shown in Table 3.

**Table 3 Pharmacokinetic parameters of compound of formula I**

| Dose (mg/kg) | Area under curve | Half-life | Residence time | Clearance rate | Apparent volume of distribution |
|---|---|---|---|---|---|
| | AUC₀₋ₜ (ng /mL*h) | T_{1/2} (h) | MRT (h) | CL (mL/min/kg) | Vz (mL/kg) |
| 1 | 221 | 2.0 | 2.6 | 75.4 | 12962 |

Conclusion: The compound of formula I has good pharmacokinetics.

### Example 2 Preparation of Hydrochloride of Compound of Formula I

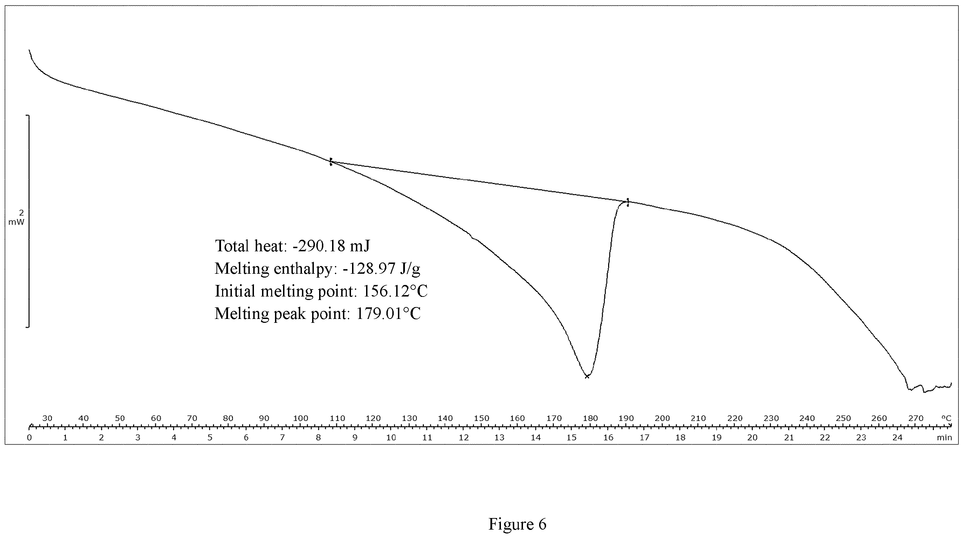

About 10 mg of the compound of formula I was weighed, then 100 µL of isopropanol was added, stirred and dissolved to clarification, and 11 µL of 2 M hydrochloric acid solution was added. The mixture was stirred for crystallization, centrifuged, and dried under vacuum to obtain the product. After X-ray powder diffraction detection, the product was defined as crystal form A. The XRPD pattern is shown in Figure 2, and its characteristic peak positions are shown in Table 4. The content of chloride ions was determined by ion chromatography to be 6.22%. The DSC pattern is shown in Figure 6.

**Table 4 XRD characteristic peak positions of crystal form A of hydrochloride**

| Peak number | 2θ[°] | d[Å] | I[%] |
|---|---|---|---|
| Peak 1 | 10.257 | 8.61711 | 17.9 |
| Peak 2 | 10.560 | 8.37085 | 70.1 |
| Peak 3 | 10.966 | 8.06140 | 25.7 |
| Peak 4 | 11.414 | 7.74651 | 100.0 |
| Peak 5 | 12.341 | 7.16651 | 23.8 |
| Peak 6 | 13.637 | 6.48812 | 49.5 |
| Peak 7 | 14.290 | 6.19309 | 21.9 |
| Peak 8 | 15.045 | 5.88383 | 38.1 |
| Peak 9 | 17.136 | 5.17033 | 30.8 |
| Peak 10 | 17.642 | 5.02316 | 49.5 |
| Peak 11 | 18.163 | 4.88018 | 72.6 |
| Peak 12 | 18.792 | 4.71819 | 66.5 |
| Peak 13 | 20.153 | 4.40254 | 22.0 |
| Peak 14 | 20.484 | 4.33233 | 63.9 |
| Peak 15 | 21.264 | 4.17514 | 26.8 |
| Peak 16 | 22.048 | 4.02833 | 61.1 |
| Peak 17 | 22.979 | 3.86724 | 20.6 |
| Peak 18 | 23.683 | 3.75378 | 9.6 |
| Peak 19 | 24.443 | 3.63880 | 22.2 |
| Peak 20 | 25.003 | 3.55859 | 10.1 |
| Peak 21 | 25.434 | 3.49925 | 28.2 |
| Peak 22 | 26.058 | 3.41678 | 6.4 |
| Peak 23 | 26.591 | 3.34954 | 16.2 |
| Peak 24 | 27.015 | 3.29792 | 6.4 |
| Peak 25 | 27.525 | 3.23796 | 63.1 |
| Peak 26 | 28.301 | 3.15086 | 5.7 |
| Peak 27 | 28.730 | 3.10479 | 15.8 |
| Peak 28 | 29.439 | 3.03167 | 24.0 |
| Peak 29 | 29.819 | 2.99388 | 5.7 |
| Peak 30 | 30.116 | 2.96504 | 6.2 |
| Peak 31 | 30.418 | 2.93625 | 20.1 |
| Peak 32 | 31.440 | 2.84309 | 15.3 |
| Peak 33 | 31.897 | 2.80340 | 6.1 |
| Peak 34 | 32.194 | 2.77822 | 5.7 |
| Peak 35 | 32.689 | 2.73729 | 1.9 |
| Peak 36 | 33.315 | 2.68721 | 14.3 |
| Peak 37 | 34.878 | 2.57031 | 27.7 |
| Peak 38 | 35.724 | 2.51139 | 1.8 |
| Peak 39 | 36.878 | 2.43538 | 1.6 |
| Peak 40 | 37.307 | 2.40836 | 0.9 |
| Peak 41 | 38.099 | 2.36012 | 4.6 |
| Peak 42 | 39.880 | 2.25871 | 5.4 |
| Peak 43 | 40.969 | 2.20117 | 3.9 |

### Example 3 Preparation of Hydrochloride of Compound of Formula I

About 10 mg of the compound of formula I was weighed, then 100 µL of acetonitrile was added, stirred and dissolved to clarification, and 11 µL of 2 M hydrochloric acid solution was added. The mixture was stirred for crystallization, centrifuged, and dried under vacuum to obtain the product. The product was detected as crystal form A by X-ray powder diffraction.

### Example 4 Preparation of Hydrochloride of Compound of Formula I

About 10 mg of the compound of formula I was weighed, then 100 µL of 7% water/ethanol was added, stirred and dissolved to clarification, and then 11 µL of 2 M hydrochloric acid solution was added. The mixture was stirred for crystallization, centrifuged, and dried under vacuum to obtain the product. The product was detected as crystal form A by X-ray powder diffraction.

### Example 5 Preparation of Hydrochloride of Compound of Formula I

About 300 mg of the compound of formula I was weighed, then 3 mL of isopropanol was added, stirred and dissolved to clarification, then 330 µL of 2 M hydrochloric acid solution was added, and 10 mL of isopropyl acetate was added. The mixture was stirred for crystallization, centrifuged, and dried under vacuum to obtain the product. The product was detected as crystal form A by X-ray powder diffraction.

### Example 6 Preparation of Methanesulfonate of Compound of Formula I

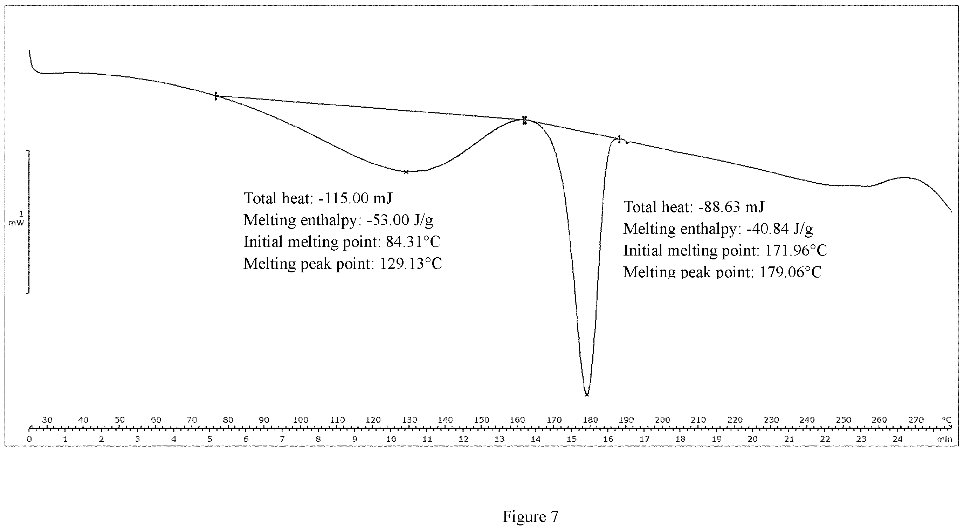

About 10 mg of the compound of formula I was weighed, then 100 µL of isopropanol was added, stirred and dissolved to clarification, then 11 µL of 2 M methanesulfonic acid solution was added, and 600 µL of isopropyl acetate was added. The mixture was stirred for crystallization, centrifuged, and dried under vacuum to obtain the product. After X-ray powder diffraction detection, the product was defined as crystal form A. The XRPD pattern is shown in Figure 3, and its characteristic peak positions are shown in Table 5. The content of methanesulfonic acid was determined by ion chromatography to be 15.47%. The DSC pattern is shown in Figure 7.

**Table 5 XRD characteristic peak positions of crystal form A of methanesulfonate**

| Peak number | 2θ[°] | d[Å] | I[%] |
|---|---|---|---|
| Peak 1 | 9.052 | 9.76141 | 31.5 |
| Peak 2 | 9.175 | 9.63067 | 30.7 |
| Peak 3 | 10.079 | 8.76916 | 11.8 |
| Peak 4 | 10.951 | 8.07284 | 79.5 |
| Peak 5 | 12.162 | 7.27152 | 18.6 |
| Peak 6 | 13.619 | 6.49670 | 29.2 |
| Peak 7 | 14.938 | 5.92602 | 72.9 |
| Peak 8 | 15.363 | 5.76302 | 28.7 |
| Peak 9 | 15.821 | 5.59693 | 12.2 |
| Peak 10 | 16.171 | 5.47670 | 32.0 |
| Peak 11 | 17.010 | 5.20840 | 100.0 |
| Peak 12 | 17.950 | 4.93768 | 21.1 |
| Peak 13 | 18.620 | 4.76160 | 11.0 |
| Peak 14 | 19.257 | 4.60549 | 29.2 |
| Peak 15 | 20.145 | 4.40447 | 82.3 |
| Peak 16 | 20.914 | 4.24406 | 49.5 |
| Peak 17 | 21.239 | 4.17992 | 49.4 |
| Peak 18 | 22.194 | 4.00217 | 24.7 |
| Peak 19 | 23.138 | 3.84101 | 5.3 |
| Peak 20 | 23.563 | 3.77271 | 28.2 |
| Peak 21 | 24.250 | 3.66724 | 32.1 |
| Peak 22 | 24.508 | 3.62930 | 25.8 |
| Peak 23 | 24.887 | 3.57489 | 15.0 |
| Peak 24 | 25.003 | 3.55849 | 15.4 |
| Peak 25 | 25.207 | 3.53014 | 18.8 |
| Peak 26 | 25.965 | 3.42880 | 5.5 |
| Peak 27 | 27.355 | 3.25765 | 30.7 |
| Peak 28 | 27.743 | 3.21295 | 9.1 |
| Peak 29 | 28.793 | 3.09819 | 8.7 |
| Peak 30 | 29.358 | 3.03977 | 23.2 |
| Peak 31 | 29.784 | 2.99731 | 7.6 |
| Peak 32 | 30.221 | 2.95494 | 2.5 |
| Peak 33 | 30.775 | 2.90301 | 7.9 |
| Peak 34 | 31.278 | 2.85742 | 12.8 |
| Peak 35 | 32.057 | 2.78973 | 3.9 |
| Peak 36 | 32.786 | 2.72937 | 8.6 |
| Peak 37 | 33.836 | 2.64708 | 4.9 |
| Peak 38 | 34.448 | 2.60143 | 0.2 |
| Peak 39 | 35.526 | 2.52489 | 2.6 |
| Peak 40 | 37.858 | 2.37455 | 3.9 |
| Peak 41 | 41.094 | 2.19475 | 2.3 |
| Peak 42 | 42.376 | 2.13124 | 2.5 |
| Peak 43 | 42.580 | 2.12150 | 3.9 |

### Example 7 Preparation of Methanesulfonate of Compound of Formula I

About 10 mg of the compound of formula I was weighed, then 100 µL of acetonitrile was added, stirred and dissolved to clarification, then 11 µL of 2 M methanesulfonic acid solution was added, and 600 µL of isopropyl acetate was added. The mixture was stirred for crystallization, centrifuged, and dried under vacuum to obtain the product. The product was detected as crystal form A by X-ray powder diffraction.

### Example 8 Preparation of Methanesulfonate of Compound of Formula I

About 10 mg of the compound of formula I was weighed, then 100 µL of 7% water/ethanol was added, stirred and dissolved to clarification, then 11 µL of 2 M methanesulfonic acid solution was added, and 600 µL of isopropyl acetate was added. The mixture was stirred for crystallization, centrifuged, and dried under vacuum to obtain the product. The product was detected as crystal form A by X-ray powder diffraction.

### Example 9 Preparation of Methanesulfonate of Compound of Formula I

About 4 g of the compound of formula I was weighed, then 16 mL of ethanol was added, stirred and dissolved to clarification, then 4.4 mL of 2 M methanesulfonic acid solution was added, and 120 mL of isopropyl acetate was added. The mixture was stirred for crystallization at room temperature, centrifuged, and dried under vacuum to obtain the product. The product was detected as crystal form A by X-ray powder diffraction.

### Example 10 Preparation of Acetate of Compound of Formula I

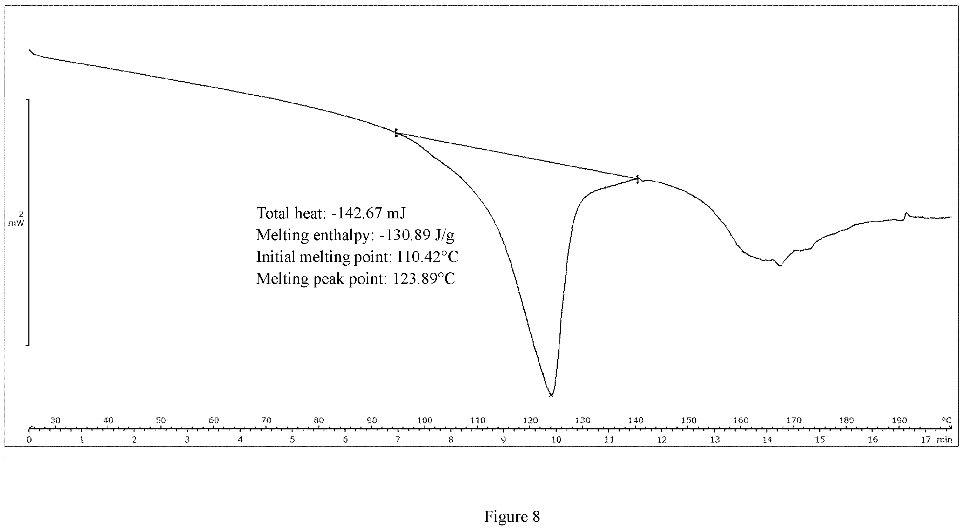

About 10 mg of the compound of formula I was weighed, then 100 µL of isopropanol was added, stirred and dissolved to clarification, and 11 µL of 2 M acetic acid solution was added. The mixture was stirred at 50°C for 1 h, then slowly cooled to 5°C to precipitate a solid, centrifuged, and dried under vacuum to obtain the product. After X-ray powder diffraction detection, the product was defined as crystal form α. The XRPD pattern is shown in Figure 4, and its characteristic peak positions are shown in Table 6. The content of acetic acid was determined by ion chromatography to be 10.26%. The DSC pattern is shown in Figure 8.

**Table 6 XRD characteristic peak positions of crystal form α of acetate**

| Peak number | 2θ[°] | d[Å] | I[%] |
|---|---|---|---|
| Peak 1 | 8.889 | 9.94015 | 100.0 |
| Peak 2 | 10.319 | 8.56530 | 6.3 |
| Peak 3 | 10.974 | 8.05601 | 60.3 |
| Peak 4 | 13.580 | 6.51501 | 14.1 |
| Peak 5 | 14.861 | 5.95653 | 24.9 |
| Peak 6 | 15.250 | 5.80522 | 5.2 |
| Peak 7 | 15.669 | 5.65093 | 38.5 |
| Peak 8 | 16.231 | 5.45643 | 1.3 |
| Peak 9 | 16.982 | 5.21691 | 38.4 |
| Peak 10 | 18.139 | 4.88658 | 2.8 |
| Peak 11 | 18.643 | 4.75558 | 15.6 |
| Peak 12 | 19.092 | 4.64489 | 20.3 |
| Peak 13 | 19.749 | 4.49170 | 43.5 |
| Peak 14 | 20.365 | 4.35732 | 41.9 |
| Peak 15 | 20.776 | 4.27198 | 17.9 |
| Peak 16 | 21.250 | 4.17780 | 10.9 |
| Peak 17 | 22.152 | 4.00959 | 5.3 |
| Peak 18 | 22.628 | 3.92646 | 1.2 |
| Peak 19 | 23.624 | 3.76300 | 30.3 |
| Peak 20 | 23.931 | 3.71540 | 20.3 |
| Peak 21 | 24.930 | 3.56873 | 15.2 |
| Peak 22 | 25.976 | 3.42742 | 6.7 |
| Peak 23 | 27.225 | 3.27289 | 35.0 |
| Peak 24 | 27.897 | 3.19560 | 4.5 |
| Peak 25 | 28.279 | 3.15334 | 4.2 |
| Peak 26 | 28.660 | 3.11222 | 6.1 |
| Peak 27 | 29.047 | 3.07165 | 20.1 |
| Peak 28 | 30.786 | 2.90197 | 3.9 |
| Peak 29 | 31.113 | 2.87220 | 2.5 |
| Peak 30 | 31.686 | 2.82161 | 1.8 |
| Peak 31 | 32.401 | 2.76097 | 8.7 |
| Peak 32 | 32.721 | 2.73463 | 0.9 |
| Peak 33 | 33.594 | 2.66560 | 1.9 |
| Peak 34 | 33.921 | 2.64064 | 3.4 |
| Peak 35 | 34.248 | 2.61616 | 1.8 |
| Peak 36 | 34.548 | 2.59414 | 2.4 |
| Peak 37 | 34.766 | 2.57837 | 1.8 |
| Peak 38 | 35.338 | 2.53791 | 3.4 |
| Peak 39 | 35.774 | 2.50796 | 0.5 |
| Peak 40 | 36.374 | 2.46798 | 1.9 |
| Peak 41 | 37.028 | 2.42587 | 0.7 |
| Peak 42 | 37.437 | 2.40031 | 0.9 |
| Peak 43 | 38.418 | 2.34123 | 2.2 |
| Peak 44 | 40.462 | 2.22754 | 0.8 |

### Example 11 Preparation of Acetate of Compound of Formula I

About 10 mg of the compound of formula I was weighed, then 100 µL of acetonitrile was added, stirred and dissolved to clarification, and 11 µL of 2 M acetic acid solution was added. The mixture was stirred at 50°C for 1 h, then slowly cooled to 5°C to precipitate a solid, centrifuged, and dried under vacuum to obtain the product. The product was detected as crystal form α by X-ray powder diffraction.

### Example 12 Preparation of Acetate of Compound of Formula I

About 10 mg of the compound of formula I was weighed, then 100 µL of 7% water/ethanol was added, stirred and dissolved to clarification, and 11 µL of 2 M acetic acid solution was added. The mixture was stirred at 50°C for 1 h, then slowly cooled to 5°C to precipitate a solid, centrifuged, and dried under vacuum to obtain the product. The product was detected as crystal form α by X-ray powder diffraction.

### Example 13 Preparation of Tartrate of Compound of Formula I

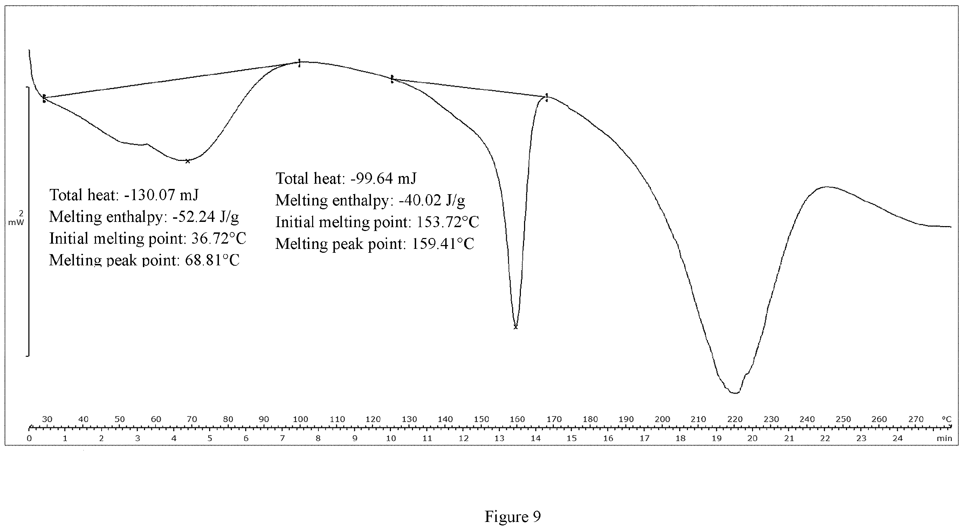

About 10 mg of the compound of formula I was weighed, then 100 µL of isopropanol was added, stirred and dissolved to clarification, and 11 µL of 2 M tartaric acid solution was added. The mixture was stirred at 50°C for 1 h, then slowly cooled to 5°C to precipitate a solid, centrifuged, and dried under vacuum to obtain the product. After X-ray powder diffraction detection, the product was defined as crystal form I. The XRPD pattern is shown in Figure 5, and its characteristic peak positions are shown in Table 7. The DSC pattern is shown in Figure 9.

**Table 7 XRD characteristic peak positions of crystal form I of tartrate**

| Peak number | 2θ[°] | d[Å] | 1[%] |
|---|---|---|---|
| Peak 1 | 7.860 | 11.23911 | 13.2 |
| Peak 2 | 8.608 | 10.26420 | 100.0 |
| Peak 3 | 9.960 | 8.87328 | 76.2 |
| Peak 4 | 10.884 | 8.12226 | 27.0 |
| Peak 5 | 11.709 | 7.55196 | 44.2 |
| Peak 6 | 13.946 | 6.34509 | 12.4 |
| Peak 7 | 14.178 | 6.24184 | 10.2 |
| Peak 8 | 14.390 | 6.15014 | 10.4 |
| Peak 9 | 15.337 | 5.77245 | 11.5 |
| Peak 10 | 15.781 | 5.61101 | 4.2 |
| Peak 11 | 16.360 | 5.41386 | 29.0 |
| Peak 12 | 17.350 | 5.10722 | 25.6 |
| Peak 13 | 17.501 | 5.06339 | 25.2 |
| Peak 14 | 17.778 | 4.98498 | 73.1 |
| Peak 15 | 18.158 | 4.88167 | 20.7 |
| Peak 16 | 18.966 | 4.67544 | 3.0 |
| Peak 17 | 20.087 | 4.41685 | 9.2 |
| Peak 18 | 21.176 | 4.19219 | 12.6 |
| Peak 19 | 21.506 | 4.12863 | 18.1 |
| Peak 20 | 22.298 | 3.98379 | 34.0 |
| Peak 21 | 23.353 | 3.80606 | 6.5 |
| Peak 22 | 23.650 | 3.75894 | 12.9 |
| Peak 23 | 23.980 | 3.70798 | 17.1 |
| Peak 24 | 24.640 | 3.61017 | 9.5 |
| Peak 25 | 25.135 | 3.54021 | 10.1 |
| Peak 26 | 26.256 | 3.39147 | 16.2 |
| Peak 27 | 27.545 | 3.23558 | 2.3 |
| Peak 28 | 27.741 | 3.21327 | 2.8 |
| Peak 29 | 28.697 | 3.10829 | 9.5 |
| Peak 30 | 29.291 | 3.04661 | 4.8 |
| Peak 31 | 30.182 | 2.95871 | 2.7 |
| Peak 32 | 30.643 | 2.91516 | 7.8 |
| Peak 33 | 30.940 | 2.88786 | 6.1 |

### Example 14 Preparation of Tartrate of Compound of Formula I

About 10 mg of the compound of formula I was weighed, then 100 µL of 7% water/ethanol was added, stirred and dissolved to clarification, and 11 µL of 2 M tartaric acid solution was added. The mixture was stirred at 50°C for 1 h, then slowly cooled to 5°C to precipitate a solid, centrifuged, and dried under vacuum to obtain the product. The product was detected as crystal form I by X-ray powder diffraction.

### Example 15 Study on the Hygroscopicity of Crystal Form A of Hydrochloride, Crystal Form A of Methanesulfonate, Crystal Form α of Acetate, and Crystal Form I of Tartrate

Using Surface Measurement Systems advantage 2, starting at a humidity of 50% at 25°C, the humidity range was 0%-95%, the step was 10%, the judgment standard was that the mass change dM/dT of each gradient was less than 0.002%, the running time TMAX of each humidity gradient was 360 min, with two cycles, and the specific results are shown in Table 8.

**Table 8**

| Test sample | 0.0%RH-95.0%RH | 0%RH-80.0%RH | Crystal form |
|---|---|---|---|
| Crystal form A of hydrochloride | 0.79% | 0.60% (slightly hygroscopic) | Untransformed |
| Crystal form A of methanesulfonate | 0.43% | 0.31% (slightly hygroscopic) | Untransformed |
| Crystal form α of acetate | 0.66% | 0.50% (slightly hygroscopic) | Untransformed |
| Crystal form I of tartrate | 18.02% | 8.82% (hygroscopic) | Untransformed |

### Example 16 Study on Stability of Crystal Form

The crystal form A of hydrochloride, the crystal form A of methanesulfonate, and the crystal form α of the acetate were placed flat and left open. The stability of the samples was investigated under conditions of illumination (4500 Lux), high temperature (40°C, 60°C), and high humidity (RH 75%, RH 92.5%), and the sampling investigation period was 30 days. The results are shown in Table 9.

**Table 9**

| Condition | Time (days) | Crystal form A of hydrochloride | |
|---|---|---|---|
| | | Purity % | Crystal form |
| Onset | 0 | 99.3 | A |
| 40°C | 5 | 99.3 | Untransformed |
| | 10 | 99.3 | Untransformed |
| | 30 | 99.3 | Untransformed |
| 60°C | 5 | 99.3 | Untransformed |
| | 10 | 99.3 | Untransformed |
| | 30 | 99.2 | Untransformed |
| 75% RH | 5 | 99.3 | Untransformed |
| | 10 | 99.3 | Untransformed |
| | 30 | 99.4 | Untransformed |
| 92.5% RH | 5 | 99.3 | Untransformed |
| | 10 | 99.3 | Untransformed |
| | 30 | 99.3 | Untransformed |
| 4500 Lux | 5 | 99.3 | Untransformed |
| | 10 | 99.3 | Untransformed |
| | 30 | 99.3 | Untransformed |

| Condition | Time (days) | Crystal form A of methanesulfonate | |
|---|---|---|---|
| | | Purity % | Crystal form |
| Onset | 0 | 99.9 | A |
| 40°C | 5 | 99.9 | Untransformed |
| | 10 | 99.9 | Untransformed |
| | 30 | 99.9 | Untransformed |
| 60°C | 5 | 99.9 | Untransformed |
| | 10 | 99.9 | Untransformed |
| | 30 | 99.9 | Untransformed |
| 75% RH | 5 | 99.9 | Untransformed |
| | 10 | 99.9 | Untransformed |
| | 30 | 99.9 | Untransformed |
| 92.5% RH | 5 | 99.9 | Untransformed |
| | 10 | 99.9 | Untransformed |
| | 30 | 99.9 | Untransformed |
| 4500 Lux | 5 | 99.9 | Untransformed |
| | 10 | 99.9 | Untransformed |
| | 30 | 99.9 | Untransformed |

| Condition | Time (days) | Crystal form α of acetate | |
|---|---|---|---|
| | | Purity % | Crystal form |
| Onset | 0 | 99.7 | α |
| 40°C | 5 | 99.6 | Untransformed |
| | 10 | 99.6 | Untransformed |
| | 30 | 99.0 | Untransformed |
| 60°C | 5 | 99.6 | Untransformed |
| | 10 | 99.5 | Untransformed |
| | 30 | 98.8 | Untransformed |
| 75% RH | 5 | 99.7 | Untransformed |
| | 10 | 99.7 | Untransformed |
| | 30 | 99.7 | Untransformed |
| 92.5% RH | 5 | 99.7 | Untransformed |
| | 10 | 99.7 | Untransformed |
| | 30 | 99.7 | Untransformed |
| 4500 Lux | 5 | 99.7 | Untransformed |
| | 10 | 99.7 | Untransformed |
| | 30 | 99.6 | Untransformed |

Conclusion: The influencing factor experiment shows that after being placed under the conditions of illumination, high temperature of 40°C and 60°C, high humidity of 75% and 92.5% for 30 days, the crystal form A of hydrochloride and the crystal form A of methanesulfonate have good physical and chemical stability; the crystal form α of acetate has good physical stability and good chemical stability under high humidity and illumination conditions.

### Example 17 Long-Term/Accelerated Stability Study

The crystal form A of hydrochloride, the crystal form A of methanesulfonate, and the crystal form α of acetate were placed at 25°C/60% RH and 40°C/75% RH to investigate the stability, and the results are shown in Tables 10, 11, and 12 below.

**Table 10 Long-term/accelerated stability of crystal form A of hydrochloride**

| Sample | Placement conditions | Purity % | Purity % | Purity % | Purity % | Purity % | Crystal form |
|---|---|---|---|---|---|---|---|
| | | Onset | 1 month | 2 month | 3 month | 6 month | |
| Crystal form A of hydrochloride | 25°C/60% RH | 99.3 | 99.3 | 99.3 | 99.2 | 99.3 | Untransformed |
| | 40°C/75% RH | 99.3 | 99.3 | 99.3 | 99.2 | 99.3 | Untransformed |

**Table 11 Long-term/accelerated stability of crystal form A of methanesulfonate**

| Sample | Placement | Purity | Purity | Purity | Purity | Purity | Crystal form |
|---|---|---|---|---|---|---|---|
| | conditions | % | % | % | % | % | |
| | | Onset | 1 month | 2 month | 3 month | 6 month | |
| Crystal form A of methanesulfonat e | 25°C/60%R H | 99.9 | 99.9 | 99.8 | 99.8 | 99.9 | Untransforme d |
| | 40°C/75%R H | 99.9 | 99.9 | 99.8 | 99.8 | 99.8 | Untransforme d |
| **[0185]** Table 12 Long-term/accelerated stability of crystal form α of acetate | | | | | | | |
| Sample | Placement conditions Onset | % Purity | % Purity | Purity % | Purity % | Purity % | Crystal form |
| | | 1 | month 2 | month 3 | month | | 6 month |
| Crystal form α of acetate | 25°C/60%RH 99.7 | | 99.7 | 99.7 | 99.7 | 99.7 | Untransformed |
| | 40°C/75%RH 99.7 | | 99.7 | 99.6 | 99.6 | 99.5 | Untransformed |

Conclusion: Long-term/accelerated experiments show the good physical and chemical stability of the crystal form A of hydrochloride, the crystal form A of methanesulfonate, and the crystal form α of acetate after being placed at 25°C/60RH and 40°C/75RH for 6 months.

## Claims

1. A pharmaceutically acceptable salt of a compound of formula I, wherein the pharmaceutically acceptable salt is selected from hydrochloride, methanesulfonate, acetate, or tartrate,

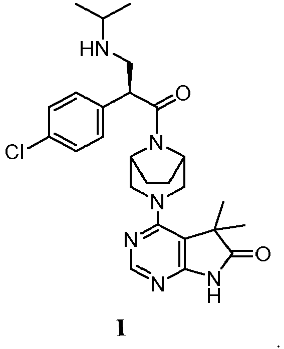

2. The pharmaceutically acceptable salt of the compound of formula I according to claim 1, wherein the molar ratio of the compound of formula I to the acid radical is selected from 1:2 to 3:1, preferably 1:1 and 2:1.

3. A crystal form A of hydrochloride of a compound of formula I, which has an X-ray powder diffraction pattern comprising characteristic peaks at 2θ angles of 10.6, 11.4, 13.6, 17.6, 18.2, and 18.8.

4. The crystal form A of the hydrochloride of the compound of formula I according to claim 3, which has the X-ray powder diffraction pattern comprising characteristic peaks at 2θ angles of 10.6, 11.4, 13.6, 15.0, 17.1, 17.6, 18.2, 18.8, 20.5, 22.0, and 27.5.

5. The crystal form A of the hydrochloride of the compound of formula I according to claim 3, which has the X-ray powder diffraction pattern comprising characteristic peaks at 2θ angles of 10.6, 11.0, 11.4, 12.3, 13.6, 14.3, 15.0, 17.1, 17.6, 18.2, 18.8, 20.2, 20.5, 21.3, 22.0, 23.0, 23.7, 24.4, 25.0, 25.4, 26.1, 26.6, 27.5, 28.3, 28.7, 29.4, 30.4, 31.4, 31.9, 32.2, 33.3, 34.9, 38.1, 39.9, and 41.0.

6. The crystal form A of the hydrochloride of the compound of formula I according to claim 3, which has the X-ray powder diffraction pattern as shown in Figure 2.

7. A crystal form A of methanesulfonate of a compound of formula I, which has an X-ray powder diffraction pattern comprising characteristic peaks at 2θ angles of 9.1, 11.0, 14.9, 16.2, and 17.0.

8. The crystal form A of the methanesulfonate of the compound of formula I according to claim 7, which has the X-ray powder diffraction pattern comprising characteristic peaks at 2θ angles of 9.1, 11.0, 14.9, 16.2, 17.0, 20.1, 20.9, 21.2, 24.3, and 27.4.

9. The crystal form A of the methanesulfonate of the compound of formula I according to claim 7, which has the X-ray powder diffraction pattern comprising characteristic peaks at 2θ angles of 9.1, 10.1, 11.0, 12.2, 13.6, 14.9, 15.4, 15.8, 16.2, 17.0, 18.0, 18.6, 19.3, 20.1, 20.9, 21.2, 22.2, 23.1, 23.6, 24.3, 24.5, 24.9, 25.0, 25.2, 26.0, 27.4, 27.7, 28.8, 29.4, 29.8, 30.2, 30.8, 31.3, 32.1, 32.8, and 33.8.

10. The crystal form A of the methanesulfonate of the compound of formula I according to claim 7, which has the X-ray powder diffraction pattern as shown in Figure 3.

11. A crystal form α of acetate of a compound of formula I, which has an X-ray powder diffraction pattern comprising characteristic peaks at 2θ angles of 8.9, 11.0, 14.9, 15.7, 17.0, and 19.7.

12. The crystal form α of the acetate of the compound of formula I according to claim 11, which has the X-ray powder diffraction pattern comprising characteristic peaks at 2θ angles of 8.9, 11.0, 14.9, 15.7, 17.0, 19.1, 19.7, 20.4, 23.6, 23.9, and 27.2.

13. The crystal form α of the acetate of the compound of formula I according to claim 11, which has the X-ray powder diffraction pattern comprising characteristic peaks at 2θ angles of 8.9, 10.3, 11.0, 13.6, 14.9, 15.7, 17.0, 18.1, 18.6, 19.1, 19.7, 20.4, 20.8, 21.3, 22.2, 23.6, 23.9, 24.9, 26.0, 27.2, 28.3, 28.7, 29.0, 30.8, 32.4, 33.6, 33.9, 34.5, 35.3, 36.4, and 38.4.

14. The crystal form α of the acetate of the compound of formula I according to claim 11, which has the X-ray powder diffraction pattern as shown in Figure 4.

15. A crystal form I of tartrate of a compound of formula I, which has an X-ray powder diffraction pattern comprising characteristic peaks at 7.9, 8.6, 10.0, 10.9, 11.7, 13.9, 14.2, 14.4, 15.3, 16.4, 17.4, 17.8, 19.0, 20.1, 21.2, 21.5, 22.3, 23.7, 24.0, 24.6, 25.1, 26.3, 27.7, 28.7, 29.3, 30.6, and 30.9.

16. The crystal form I of the tartrate of the compound of formula I according to claim 15, which has the X-ray powder diffraction pattern as shown in Figure 5.

17. The crystal form of the pharmaceutically acceptable salt of the compound of formula I according to any one of claims 3 to 16, wherein the 2θ angle has an error range of ± 0.2.

18. A method for preparing the crystal form A of the hydrochloride of the compound of formula I according to any one of claims 3 to 6, the method comprises: mixing the compound of formula I with an appropriate amount of a solvent and hydrochloric acid, and stirring for crystallization, the solvent being one or more than one of isopropanol, acetonitrile, water/ethanol, and isopropyl acetate.

19. A method for preparing the crystal form A of the methanesulfonate of the compound of formula I according to any one of claims 7 to 10, the method comprises: mixing the compound of formula I with an appropriate amount of a solvent and methanesulfonic acid, and stirring for crystallization, the solvent being one or more than one of isopropanol, acetonitrile, ethanol, water/ethanol, and isopropyl acetate.

20. A method for preparing the crystal form α of the acetate of the compound of formula I according to any one of claims 11 to 14, the method comprises: mixing the compound of formula I with an appropriate amount of a solvent and acetic acid, and stirring for crystallization, the solvent being one or more than one of isopropanol, acetonitrile, and water/ethanol.

21. A method for preparing the crystal form I of the tartrate of the compound of formula I according to any one of claims 15 to 16, the method comprises: mixing the compound of formula I with an appropriate amount of a solvent and tartaric acid, and stirring for crystallization, the solvent being one or more than one of isopropanol and water/ethanol.

22. A pharmaceutical composition comprising the following components:
(a) the pharmaceutically acceptable salt of the compound of formula I according to any one of claims 1 to 2 or the crystal form of the pharmaceutically acceptable salt of the compound of formula I according to any one of claims 3 to 17; and
(b) an optional pharmaceutically acceptable carrier, diluent, or excipient.

23. A method for preparing a pharmaceutical composition, comprising a step of mixing:
(a) the pharmaceutically acceptable salt of the compound of formula I according to any one of claims 1 to 2 or the crystal form of the pharmaceutically acceptable salt of the compound of formula I according to any one of claims 3 to 17; and
(b) an optional pharmaceutically acceptable carrier, diluent, or excipient.

24. A use of the pharmaceutically acceptable salt of the compound of formula I according to claim 1 or 2, the crystal form of the pharmaceutically acceptable salt of the compound of formula I according to any one of claims 3 to 17, or the composition according to claim 22 in the manufacture of a medicament for treating and/or preventing cancer, wherein the cancer is preferably selected from ovarian cancer, breast cancer, prostate cancer, neuroglioma, spongiocytoma, gastric cancer, fallopian tube cancer, lung cancer, peritoneal tumor, melanoma, brain cancer, esophageal cancer, liver cancer, pancreatic cancer, colorectal cancer, lung cancer, kidney cancer, cervical cancer, skin cancer, neuroblastoma, sarcoma, bone cancer, uterine cancer, endometrial cancer, head and neck tumor, multiple myeloma, lymphoma, non-Hodgkin's lymphoma, non-small cell lung cancer, polycythemia vera, leukemia, thyroid tumor, bladder cancer, and gallbladder cancer.
